# EUROPÄISCHE PATENTSCHRIFT

(11) **EP 2 821 051 B1**
(45) Veröffentlichungstag und Bekanntmachung des Hinweises auf die Patenterteilung: **02.11.2016**
(21) Anmeldenummer: 14157691.8
(22) Anmeldetag: 04.03.2014
(51) Int. Cl.: A61K 8/24, A61Q 11/00, A61K 8/73

(54) **Remineralisierende Mund- und Zahnpflege- und Reinigungsmittel mit Zahnfleischstimulation**
Remineralising oral and dental hygiene and cleaning agent with gum stimulation
Produit de soin et de nettoyage de la bouche et des dents reminéralisant avec stimulation gingivale

(30) Priorität: 02.07.2013 DE 102013212875
(43) Veröffentlichungstag der Anmeldung: 07.01.2015
(73) Patentinhaber: Henkel AG & Co. KGaA, 40589 Düsseldorf (DE)
(72) Erfinder: Welß, Thomas, 40591 Düsseldorf (DE); Duschek, Nicole, 40595 Düsseldorf (DE); Howorka, Wilfried, 40593 Düsseldorf (DE); Evening, Jennifer, 40472 Düsseldorf (DE)

(56) Entgegenhaltungen:
- US-A1- 2006 134 019
- US-A1- 2006 134 020
- US-A1- 2006 286 044
- US-A1- 2008 166 307

## Beschreibung

Die Erfindung betrifft Zubereitungen zur Mund- und Zahnpflege- und -reinigung, die aufgrund von speziellen Inhaltsstoffen remineralisierend wirken, eine hohe Reinigungsleistung besitzen, ohne die Zahnoberfläche zu zerkratzen oder zu sehr abrasiv zu wirken bei gleichzeitiger Vitalisierung von Zahnfleisch und Mundschleimhaut.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen.

Zahnpasten entfalten ihre Reinigungsleistung hauptsächlich durch die in der Zahnpasta enthaltenen Poliermittel, in geringerem Maße auch durch die enthaltenen Tenside. Die Poliermittel müssen, um ihre Reinigungs- und Polierwirkung zu entfalten, eine gewisse Abrasivität gegenüber der Zahnoberfläche aufweisen. Es ist jedoch von größter Bedeutung, daß die Abrasivität gegenüber Zahnschmelz und Dentin auf niedrigen Werten gehalten wird, um eine Schädigung der Zahnoberfläche durch den täglichen Gebrauch der Zahnpasta zu vermeiden. Vor allem dürfen die verwendeten Abrasiva keinen unnötigen Abtrag des Zahnschmelzes (Enamel) bewirken, und auch das darunterliegende, weichere Dentin möglichst nicht schädigen, da bei Personen mit schmerzempfindlichen Zähnen häufig die Ursache in einer dünnen Enamelschicht oder freiliegenden Zahnhälsen liegt.

Der Mundraum ist mit einer befeuchteten Schleimhaut (Mucosa) ausgekleidet. Diese besteht generell aus zwei Lagen: einem mehrschichtigen, in der Regel unverhornten Plattenepithel und dem darunter liegenden Bindegewebe. Im Gegensatz zur normalen Haut besitzt die Schleimhaut keine Hornschicht. Als Zahnfleisch wird der epitheliale Bestandteil des Zahnhalteapparates bezeichnet. Auch dieses besteht aus einem mehrschichtigen Plattenepithel, welches ebenfalls nur wenige Hornschichten aufweist.

Ein wichtiges Charakteristikum von Mundschleimhaut und Zahnfleisch ist demzufolge das Fehlen bzw. die sehr schwache Ausbildung der Hornschicht. Mit dieser Hornschicht fehlt der gesamten Mundregion auch eine wichtige äußere Barriere gegenüber mechanischen, chemischen und physikalischen Einflüssen. Die Schleimhautzellen sind somit allen vorgenannten Beanspruchungen ohne Schutzschicht ausgesetzt und reagieren aus diesem Grund empfindlicher auf die durch das Zähneputzen hervorgerufenen mechanischen Beanspruchungen und die mit der Anwendung von Zahncremes verbundenen chemischen Reize.

Der vorliegenden Anmeldung lag daher die Aufgabe zugrunde, ein sowohl für die Zähne als auch für das Zahnfleisch bzw. die Mundschleimhaut optimiertes Zahnreinigungs- und Pflegemittel bereitzustellen, welches den Einsatz der für Zahnreinigung und Kariesprophylaxe unverzichtbaren Inhaltsstoffe wie Abrasiva und Tenside ermöglicht, hierbei aber gleichzeitig deren negativen Einfluss auf das Zahnfleisch minimiert, das Zahnfleisch vitalisiert und dessen Widerstandsfähigkeit stärkt.

Die Zahnreinigung ist auch eine mechanische Beanspruchung, welche - abhängig von der Reinigungstechnik, der Bürstenkonstruktion und des während des Putzvorgangs auf den Bürstenkopf ausgeübten Drucks - dem Zahnfleisch bzw. der Mundschleimhaut kleine Verletzungen zufügen kann. Diese Mikroverletzungen werden vom Verbraucher oftmals gar nicht bemerkt, können aber im schlimmsten Fall zu Schmerzreizen oder Zahnfleischbluten während des Reinigungsvorgangs führen. Je vitaler die Mundschleimhaut bzw. das Zahnfleisch ist, desto widerstandsfähiger ist es gegenüber entsprechenden Mikroverletzungen, und desto schneller und komplikationsloser heilen diese Mikroverletzungen ab.

Es ist daher weiterhin die Aufgabe dieser Erfindung, Zahnreinigungsmittel bereitzustellen, welche das Zahnfleisch bereits während des Reinigungsvorgangs vitalisieren und seine Widerstandsfähigkeit stärken, so dass auf diese Weise die Entstehung kleiner Mikroverletzungen verhindert und - sollten doch kleine Verletzungen verursacht worden sein - die Regenerationsfähigkeit des Zahnfleisches erhöht wird.

Die US 2006/134020 A1, die US 2006/286044 A1, die US 2006/134019 A1 sowie die US 2008/16307 A1 offenbaren Zusammensetzungen, welche Calcium-Glycerophosphat, Xanthan und CMC enthalten. Der Einsatz von Betainen oder die Bekämpfung von Zahnfleischentzündungen oder der Behandlung von Verletzungen des lebenden Mundgewebes werden nicht adressiert.

In nicht vorhersehbarer Weise wurde nun gefunden, daß die zuvor beschriebenen Aufgaben durch eine Wirkstoffkombination aus Calcium-Glycerophosphat, Cocamidopropylbetain und zwei Polymeren gelöst werden kann. Die Polymere bewirken in Kombination mit dem Calcium-Glycerophosphat und dem Cocamidopropylbetain die Ausbildung einer mikrofeinen Schicht auf dem Zahnschmelz, welche zu einer signifikanten Reduktion der Oberflächenrauheit führt. Zudem bewirken die beiden Polymere einen stabilen und feinporigen Schaum, der die Deposition von Aktivstoffen auf den Zahnschmelz sowie auf Zahnfleisch und Mundschleimhaut intensiviert.

Gegenstand der vorliegenden Erfindung ist in einer ersten Ausführungsform ein Mund- und Zahnpflegeund -reinigungsmittel, enthaltend - bezogen auf sein Gewicht -
a) 0,001 bis 5 Gew.-% Calcium-Glycerophosphat;
b) 0,1 bis 10 Gew.-% Carboxymethylcellulose;
c) 0,1 bis 10 Gew.-% Xanthan
d) 0,05 bis 5,0 Gew.-% Cocamidopropylbetain.
c) 0,1 bis 10 Gew.-% Xanthan.

Entsprechend konzipierte Mittel reinigen die Zähne in bewährter Weise und gewährleisten eine optimale Kariesprophylaxe, üben hierbei jedoch keine nachteiligen Einflüsse auf Zahnfleisch und Mundschleimhaut aus, da sie das Zahnfleisch bzw. die Mundschleimhaut vitalisieren und deren Widerstandsfähigkeit stärken. Zudem sind Zahnfleisch und Mundschleimhaut gegenüber mechanischen Beanspruchungen, wie sie durch den Zahnreinigungsvorgang hervorgerufen werden, besser geschützt und schneller regenerationsfähig.

Unter Vitalisierung wird im Sinne der vorliegenden Erfindung eine Erhöhung der Vitalität der Zellen des Zahnfleischs und der Mundschleimhaut verstanden. Eine Erhöhung der Vitalität bedeutet eine Erhöhung der Zellüberlebensfähigkeit, welche beispielsweise nach verschiedenen Verfahren wie dem Nachweis der Zellatmungsaktivität mittels Vitalstoffen ermittelt werden kann. Die Erhöhung der Vitalität einer Zelle verbessert auch deren Widerstandsfähigkeit.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Als ersten wesentlichen Bestandteil enthalten die erfindungsgemäßen Mund und Zahnpflege- und - reinigungsmittel 0,001 bis 5 Gew.-% Calcium-Glycerophosphat, d.h. mindestens eines Calciumsalzes mindestens einer Glycerophosphorsäure.

Die Glycerophosphorsäure ist eine zweibasige Säure, die in zwei isomeren Formen vorkommt, je nachdem, ob die Phosphorsäuregruppierung an eine terminale oder die mediale OH-Gruppe des Glycerins gebunden vorliegt. Die Form, bei der die Phosphorsäuregruppierung an eine terminale OH-Gruppe des Glycerins gebunden vorliegt, wird auch als alpha-Isomer, die Form, bei der die Phosphorsäuregruppierung an die mediale OH-Gruppe des Glycerins gebunden vorliegt, auch als beta-Isomer bezeichnet.

Das alpha-Isomer ist zusätzlich optisch aktiv und tritt in den zwei enantiomeren Formen sn-Glycerol-1-phosphorsäure 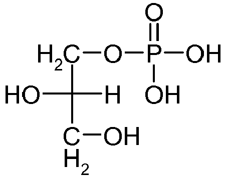 sowie sn-Glycerol-3-phosphorsäure 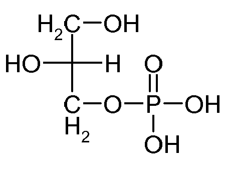 auf. Das Präfix *sn* bei Glycerol-Derivaten steht für "stereospezifisch nummeriert" und verlangt, daß die 2-Hydroxy-Gruppe in der vorstehend verwendeten Fischer-Projektion nach links weist. Glycerol-2-phosphat ist nicht optisch aktiv. Die Glycerophosphorsäuren sind etwa so stark wie Phosphorsäure. Erfindungsgemäß bevorzugt ist der Einsatz des alpha-Isomers, unabhängig davon, welches der beiden Enantiomere eingesetzt wird. Sofern der Einsatz enantiomerenreiner Verbindungen gewünscht ist, wird vorzugsweise das Calciumsalz der sn-Glycerol-3-phosphorsäure eingesetzt. Zusammenfassend sind erfindungsgemäße Mund und Zahnpflege- und -reinigungsmittel bevorzugt, die Calciumsalze der Glycerophosphorsäuren der Formeln (la) und (Ib) enthalten

HO-CH₂-CH(OH)-CH₂-OP(O)O₂²⁻ Ca²⁺ (Ia) HO-CH₂-CH(OP(O)O₂²⁻)-CH₂-OH Ca²⁺ (Ib).

Hierbei sind besonders bevorzugte erfindungsgemäße Mund und Zahnpflege- und reinigungsmittel dadurch gekennzeichnet, daß das Gewichtsverhältnis der Calciumsalze der Formeln (la) zu (Ib) oberhalb von 50:50, vorzugsweise oberhalb von 60:40, besonders bevorzugt oberhalb von 70:30 und insbesondere oberhalb 80:20 liegt.

Der Einsatz der Calcium-Glycerophosphate innerhalb engerer Mengenbereiche ist bevorzugt. Bevorzugte erfindungsgemäße Mund- und Zahnpflege- und -reinigungsmittel enthalten - bezogen auf ihr Gewicht - 0,01 bis 2,5 Gew.-%, vorzugsweise 0,05 bis 2,0 Gew.-%, besonders bevorzugt 0,1 bis 1,0 Gew.-%, weiter bevorzugt 0,11 bis 0,75 Gew.-% und insbesondere 0,12 bis 0,5 Gew.-% Calcium-Glycerophosphat.

Als zweiten wesentlichen Bestandteil enthalten die erfindungsgemäßen Mittel 0,1 bis 10,0 Gew.-% Carboxymethylcellulose (CMC).

Die in erfindungsgemäßen Mund- und Zahnpflege- und -reinigungsmitteln eingesetzte Carboxymethylcellulose wird durch chemische Umsetzung (Veretherung) von Cellulose hergestellt. Durch die Steuerung der Reaktionsbedingungen ist es möglich, den Grad der Veretherung genau einzustellen. Als Maß für den Veretherungsgrad wird der Substitutionsgrad (Abkürzung DS) angegeben. Der Substitutionsgrad gibt die durchschnittliche Anzahl der veretherten Hydroxylgruppen an einer Glucoseeinheit an. Da eine Glucoseeinheit drei Hydroxylgruppen für eine Umsetzung zur Verfügung hat, beträgt der maximal erreichbare Substitutionsgrad DS = 3.

Es hat sich gezeigt, daß die Fluoriddeposition weiter verbessert wird, wenn die eingesetzte Carboxymethylcellulose Substitutionsgrade von 0,5 bis 2,5, vorzugsweise von 0,65 bis1,45, aufweist. Entsprechende erfindungsgemäße Produkte, die CMCs der genannten Substitutionsgrade enthalten, sind erfindungsgemäß besonders bevorzugt.

Eine weitere Möglichkeit der Verbesserung der Fluoriddeposition besteht darin, den erfindungsgemäßen Mund und Zahnpflege- und -reinigungsmittel ein definiertes rheologisches Profil zu geben. In erster Linie kann dies durch den Einsatz von CMCs erfolgen, die ihrerseits definierte rheologische Profile haben.

Viskositäten von CMC-Lösungen werden beispielsweise mit Hilfe von Brookfield Viskosimetern, Rotationsviskosimetern oder Höppler Viskosimetern bestimmt. Die Viskositäten werden in Pas oder mPa·s angegeben. Je nach Methode können sich die angegebenen Viskositäten deutlich unterscheiden. Erfindungsgemäß bevorzugt ist der Einsatz von CMCs, welche in einer 2 Gew.-%igen wäßrigen Lösung bei 20°C folgende Viskositäten aufweisen:
- 1.000 bis 100.000 mPas, vorzugsweise 2.000 bis 50.000 mPas und insbesondere 5.000 bis 25.000 mPa·s mit einem Rotationsviskosimeter bei einer Scherrate von 2,55 1/s
- 5.000 bis 100.000 mPas, vorzugsweise 10.000 bis 70.000 mPas und insbesondere 35.000 bis 50.000 mPa·s mit einem Brookfield LVT Viskosimeter
- 50.000 bis 250.000 mPas, vorzugsweise 75.000 bis 200.000 mPas und insbesondere 120.000 bis 140.000 mPas mit einem Höppler Viskosimeter

Die Viskosität einer wässrigen CMC-Lösung ist demnach keine absolute Größe, sondern wird stark von den Messparametern bestimmt.

Um die Vergleichbarkeit der angegebenen Viskositäten sicherzustellen, spezifizieren wir Walocel® CRT A - Cellulose Gum und Walocel® CRT - Produkte grundsätzlich mit Brookfield Viskosimetern bei 25°C. Walocel® CRT...PV Produkte werden mit einem Rotationsviskosimeter bei 20°C spezifiziert.

Bezogen auf das anwendungsfertige erfindungsgemäße Mittel sind solche Mund- und Zahnpflege- und -reinigungsmittel bevorzugt, die bei 20°C eine Viskosität (gemessen mit Brookfield Synchro-Lectric Viskosimeter, Typ RVT mit Helipath-Stativ, Spindel 3 und 20 U/min) von 100 bis 1000 Pas (100.000 bis 1.000.000 mPas), vorzugsweise von 200 bis 750 Pas (200.000 bis 750.000 mPas), weiter bevorzugt von 350 bis 650 Pas (350.000 bis 650.000 mPas) und insbesondere von 450 bis 550 Pas (450.000 bis 550.000 mPas) aufweisen.

Die Menge an CMC, die in bevorzugten erfindungsgemäßen Mitteln eingesetzt wird, liegt typischerweise oberhalb 0,1 Gew.-% und unterhalb 10 Gew.-%. Bevorzugte erfindungsgemäße Mund- und Zahnpflege- und -reinigungsmittel sind dadurch gekennzeichnet, daß sie - bezogen auf ihr Gewicht - 0,2 bis 7,5 Gew.-%, vorzugsweise 0,25 bis 5 Gew.-%, besonders bevorzugt 0,3 bis 4 Gew.-%, weiter bevorzugt 0,4 bis 3 Gew.-%, noch weiter bevorzugt 0,45 bis 2 Gew.-% und insbesondere 0,5 bis 0,8 Gew.-% Carboxymethylcellulose enthalten.

Als dritten wesentlichen Bestandteil enthalten die erfindungsgemäßen Mittel 0,1 bis 10,0 Gew.-% Xanthan. Xanthan bzw. Xanthangummi ist ein natürlicher, nachwachsender Rohstoff und wird als ein anionisches Polysaccharid vom Bakterium *Xanthomonas campestris* ausgeschieden.

Das Molekülgewicht des eingesetzten Xanthangummis beträgt bevorzugt 2·10⁶ bis 20·10⁶ g/mol.

Als Molekülbausteine enthält Xanthangummi D-Glucose, D-Mannose, D-Glucuronsäure, Acetat und Pyruvat in einem ungefähren molaren Verhältnis von 28 zu 30 zu 20 zu 17 zu 5,1 bis 6,3. Das Polymerrückgrat des Xanthangummis bildet sich aus einer Cellulose-Kette aus β-1,4-gebundenen Glucoseeinheiten.

Xanthan enthält Struktureinheiten der folgenden Formel

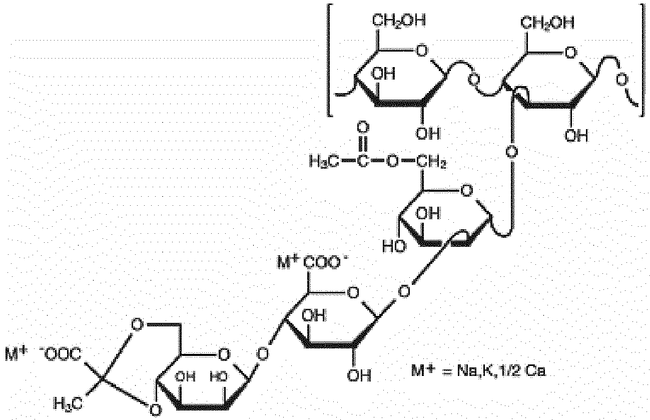

Bevorzugte erfindungsgemäße Mund- und Zahnpflege- und -reinigungsmittel enthalten - bezogen auf ihr Gewicht - 0,15 bis 5 Gew.-%, vorzugsweise 0,2 bis 2,5 Gew.-%, besonders bevorzugt 0,25 bis 1 Gew.-%, weiter bevorzugt 0,3 bis 0,75 Gew.-%, noch weiter bevorzugt 0,35 bis 0,6 Gew.-% und insbesondere 0,4 bis 0,5 Gew.-% Xanthan.

Erfindungsgemäße bevorzugt geeignete Xanthane weist in einer 1 Gew.-%-igen wässrigen KCl-Lösung eine Viskosität von mindestens 1200 bis 1600 mPa.s (Brookfield DV-I Viskosimeter, Spindel #6 bei 23°C und 10 U/min) auf.

Eine besonders effektive Kombination der beiden Polymere ist diejenige, in denen mindestens genauso viel CMC eingesetzt wird wie Xanthan. Bevorzugt wird CMC im Überschuß zu Xanthan eingesetzt, da dann besonders gute Schaumstabilitäten erzielt werden und die Mittel besonders effektiv Zahnschmelz und Zahnfleisch schützen.

Erfindungsgemäß besonders bevorzugte Mund- und Zahnpflege- und -reinigungsmittel sind dadurch gekennzeichnet, daß das Gewichtsverhältnis von Carboxymethylcellulose zu Xanthan bei ≥ 1:1, vorzugsweise bei ≥ 1,2:1, besonders bevorzugt bei ≥ 1,4:1 und insbesondere bei ≥ 1,5:1 liegt.

Als vierten wesentlichen Bestandteil enthalten die erfindungsgemäßen Mittel 0,05 bis 5,0 Gew.-% Cocamidopropylbetain.

Cocoamidopropylbetaine sind Betaine der Formel (Bet-I) 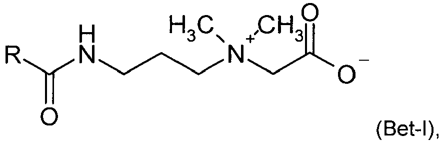 in der R für einen geradkettigen oder verzweigten, gesättigten oder ein- bzw. mehrfach ungesättigten Alkyl- oder Alkenylrest mit 8 bis 24 Kohlenstoffatomen steht und sich von Kokosfettsäuren ableitet.

Besonders bevorzugt werden erfindungsgemäß Tenside der Formel (Bet-I) eingesetzt, die ein Gemisch der folgenden Vertreter sind:

H₃C-(CH₂)₇-C(O)-NH-(CH₂)₃N⁺(CH₃)₂CH₂COO⁻

H₃C-(CH₂)₉-C(O)-NH-(CH₂)₃N⁺(CH₃)₂CH₂COO⁻

H₃C-(CH₂)₁₁-C(O)-NH-(CH₂)₃N⁺(CH₃)₂CH₂COO⁻

H₃C-(CH₂)₁₃-C(O)-NH-(CH₂)₃N⁺(CH3)₂CH₂COO⁻

H₃C-(CH₂)₁₅-C(O)-NH-(CH₂)₃N⁺(CH₃)₂CH₂COO⁻

H₃C-(CH₂)₇-CH=CH-(CH₂)₇-C(O)-NH-(CH₂)₃N⁺(CH₃)₂CH₂COO⁻

In einer weiteren besonders bevorzugten Ausführungsform ist ein erfindungsgemäßes Mund- und Zahnpflege- und -reinigungsmittel daher dadurch gekennzeichnet, daß es, bezogen auf sein Gesamtgewicht, 0,1 bis 3,5 Gew.-%, besonders bevorzugt 0,2 bis 2,5 Gew.-% und insbesondere 0,5 bis 2,0 Gew.-% Cocamidopropylbetain enthält.

Die erfindungsgemäße Kombination kann ihre vorteilhaften Wirkungen auf das Zahnfleisch und die Mundschleimhaut durch den Einsatz von Vitamin E noch weiter steigern.

Als weiteren Inhaltsstoff enthalten bevorzugte erfindungsgemäße Mund- und Zahnpflege- und -reinigungsmittel daher gegebenenfalls acetyliertes Vitamin E. Im Zuge der zu dieser Erfindung führenden Arbeiten hat sich herausgestellt, dass sich das bzw. die erfindungsgemäßen Mittel insbesondere dann besonders vitalisierend auf Zahnfleisch und Mundschleimhaut auswirken, wenn das gegebenenfalls acetylierte Vitamin E in bestimmten Konzentrationsbereichen eingesetzt wird. Mund- und Zahnpflege- und -reinigungsmittel, die, bezogen auf ihr Gesamtgewicht, 0,01 bis 1,0 Gew.-%, vorzugsweise 0,05 bis 0,5 Gew.-% gegebenenfalls acetyliertes Vitamin E enthalten, sind aus diesem Grund erfindungsgemäß bevorzugt.

Die Bezeichnung "gegebenenfalls acetyliertes Vitamin E" umfasst eine Reihe wirksamer Substanzen. Zu diesen Substanzen zählen insbesondere die Substanzfamilien der Tocopherole, der Tocotrienole sowie die Tocomonoenole und marinen Tocopherole (MDT, marine derived tocopherols).

Allen Vitamin-E-Formen gemeinsam ist ein an Position 6 hydroxyliertes Chromanring-Grundgerüst.

Die vier oben genannten Substanzfamilien unterscheiden sich hinsichtlich unterschiedlich gesättigter Seitenketten, wobei
- die Tocopherole eine gesättigte Seitenkette aufweisen;
- die Tocomonoenole und marinen Tocopherole eine einfach ungesättigte Seitenketten aufweisen; und
- die Tocotrienole eine dreifach gesättigte Seitenkette aufweisen.

In Abhängigkeit von der Methylierung des Chroman-Grundgerüsts wird in jeder der Substanzfamilien zwischen α-, β-, χ- und δ-Formen unterschieden.

So umfasst die Substanzfamilie der Tocopherole α-Tocopherol, β-Tocopherol, χ-Tocopherol, δ-Tocopherol sowie die weiteren natürlich vorkommenden Tocopherole 5,7-Dimethyltocol und 7-Methyltocol. Zur Substanzfamilie der Tocomonoenole zählt das α-Tocomonoenol, das β-Tocomonoenol, das χ-Tocomonoenol und das δ-Tocomonoenol. Die Substanzfamilie der marinen Tocopherole schließt α-MDT, β- MDT, χ- MDT und δ-MDT ein und die Substanzfamilie der Tocotrienole umfasst neben α-Tocotrienol, β-Tocotrienol, χ-Tocotrienol auch δ-Tocotrienol.

Alle vorgenannten Vitamin-E-Formen verbessern die Wirkung der erfindungsgemäßen Mittel auf Zahnfleisch und Mundschleimhaut. Aufgrund ihrer Wirkung besonders bevorzugt werden jedoch die Tocopherole, insbesondere des α-Tocopherol. Bevorzugte Mund- und Zahnpflege- und - reinigungsmittel sind dadurch gekennzeichnet, dass sie, bezogen auf ihr Gesamtgewicht, 0,001 bis 1,5 Gew.-%, vorzugsweise 0,01 bis 1,0 Gew.-%, und insbesondere 0,05 bis 0,5 Gew.-% gegebenenfalls acetyliertes α-Tocopherol enthalten.

Die Tocopherole weisen drei Stereozentren auf. Folglich existieren also von jedem der vier oben genannten Tocopherole (α-, β-, χ- und δ-Tocopherol) jeweils acht Stereoisomere. Als im Rahmen der vorliegenden Anmeldung besonders wirkungsvoll hat sich dass *RRR*-α-Tocopherol erwiesen. Besonders bevorzugte Mund- und Zahnpflege- und -reinigungsmittel sind daher dadurch gekennzeichnet, dass sie, bezogen auf ihr Gesamtgewicht, 0,001 bis 1,5 Gew.-%, vorzugsweise 0,01 bis 1,0 Gew.-%, und insbesondere 0,05 bis 0,5 Gew.-% gegebenenfalls acetyliertes *RRR*-α-Tocopherol enthalten.

Da Vitamin E einschließlich der Tocopherole und des α-Tocopherols eine vergleichsweise geringe Stabilität aufweisen, wird im Rahmen der vorliegenden Anmeldung der Einsatz von acetyliertem Tocopherol (Tocopherylacetat), beispielsweise acetyliertem α-Tocopherol, insbesondere acetyliertem *RRR-*α-Tocopherol bevorzugt. Bevorzugte Mund- und Zahnpflege- und -reinigungsmittel sind dadurch gekennzeichnet, dass sie, bezogen auf ihr Gesamtgewicht, 0,001 bis 1,5 Gew.-%, vorzugsweise 0,01 bis 1,0 Gew.-%, und insbesondere 0,05 bis 0,5 Gew.-% Tocopherylacetat, vorzugsweise α-Tocopherylacetat, insbesondere *RRR-*α-Tocopherylacetat enthalten.

Die erfindungsgemäßen Mund- und Zahnpflege- und -reinigungsmittel können weitere Inhaltsstoffe enthalten. Bevorzugt ist hierbei der Einsatz von sogenannten Feuchthaltemitteln, die bei Zahnpasten das Austrocknen verhindern. Bei sogenannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel.

Hier sind erfindungsgemäße Mund- und Zahnpflege- und -reinigungsmittel bevorzugt, die - bezogen auf ihr Gewicht - 10 bis 50 Gew.-%, vorzugsweise 12,5 bis 45 Gew.-%, besonders bevorzugt 15 bis 40 Gew.-%, weiter bevorzugt 17,5 bis 35 Gew.-% und insbesondere 20 bis 29 Gew.-% mehrwertige(n) Alkohol(e) aus der Gruppe Sorbit und/oder Glycerin und/oder 1,2-Propylenglycol oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so daß Mittel, die außer Sorbit keine anderen mehrwertigen Alkohole enthalten, besonders bevorzugt sind.

Die erfindungsgemäßen Zusammensetzungen können zusätzlich mindestens ein Poliermittel enthalten. Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels. Erfindungsgemäß bevorzugte Mittel enthalten Poliermittel innerhalb engerer Mengenbereiche. Hier sind erfindungsgemäß bevorzugte Mund- und Zahnpflege- und - reinigungsmittel dadurch gekennzeichnet, dass sie - bezogen auf ihr Gewicht - 1 bis 25 Gew.-%, vorzugsweise 2,5 bis 20 Gew.-%, weiter bevorzugt 5 bis 18 Gew.-% und insbesondere 7,5 bis 16 Gew.-% Poliermittel enthalten.

Besonders geeignet sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die so genannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, besonders geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident^{®}12 DS (DEGUSSA) erhältlich.

Andere Fällungskieselsäuren dieser Art sind Sident^{®} 8 (Evonik) und Sorbosil^{®} AC 39 (PQ Corporation). Diese Kieselsäuren zeichnen sich durch eine geringere Verdickungswirkung und eine etwas höhere mittlere Teilchengröße von 8 -14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pas aufweisen.

Zahnpasten, die eine deutlich höhere Viskosität von mehr als 100 Pas (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungs-Kieselsäuren mit einer BET-Oberfläche von 150 - 250 m²/g zu, z.B. das Handelsprodukt Sident^{®} 22S.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Erfindungsgemäß besonders bevorzugte Mund und Zahnpflege- und -reinigungsmittel enthalten ausschließlich Poliermittel aus der Gruppe der Kieselsäuren, Aluminiumhydroxid, Aluminiumoxid, Dicalciumphosphat-dihydrat (CaHPO₄ ·2H₂O) oder Gemische dieser Reibkörper. Diese Poliermittel haben sich als besonders effizient bei der Lösung der erfindungsgemäßen Aufgabe erwiesen.

Ganz besonders bevorzugt enthalten die erfindungsgemäßen Zusammensetzungen bezogen auf ihr Gewicht 1 bis 30 Gew.-% Fällungskieselsäure(n) mit einer spezifischen Oberfläche nach ISO 5794-1, Anhang D von ≤ 55 m²/g. Vorzugsweise werden die Fällungskieselsäuren, die entsprechende spezifische Oberflächen aufweisen, innerhalb engerer Mengenbereiche eingesetzt, und insbesondere bevorzugt werden Fällungskieselsäuren eingesetzt, die noch niedrigere spezifische Oberflächen nach ISO 5794-1, Anhang D aufweisen. Bevorzugte erfindungsgemäße Mund- und Zahnpflege- und - reinigungsmittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 13,0 Gew.-% Fällungskieselsäure(n) mit einer spezifischen Oberfläche nach ISO 5794-1, Anhang D von ≤ 55 m²/g. Besonders bevorzugte erfindungsgemäße Mund und Zahnpflege- und -reinigungsmittel sind dadurch gekennzeichnet, dass sämtliche im Mittel enthaltenen Fällungskieselsäure(n) eine spezifische Oberfläche nach ISO 5794-1, Anhang D von ≤ 53 m²/g, vorzugsweise von ≤ 51 m²/g, weiter bevorzugt von ≤ 49 m²/g und insbesondere von ≤ 47 m²/g aufweisen.

Als weiteren wesentlichen Inhaltsstoff können die erfindungsgemäßen Mund- und Zahnpflege- und - reinigungsmittel mindestens ein anionisches Tensid enthalten.

Geeignete anionische Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂-C₁₈)-Sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆ )-estern, Acylsarcosinen, Acyltauriden und Acylisothionaten mit jeweils 8-18 C-Atomen in der Acylgruppe.

Weitere typische Beispiele für anionische Tenside sind Seifen, Alkylbenzolsulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Erfindungsgemäß bevorzugte Mund- und Zahnpflege- und -reinigungsmittel sind dadurch gekennzeichnet, dass sie, bezogen auf ihr Gesamtgewicht, 0,05 bis 5,0 Gew.-%, vorzugsweise 0,1 bis 3,5 Gew.-%, besonders bevorzugt 0,2 bis 2,5 Gew.-% und insbesondere 0,5 bis 2,0 Gew.-% anionische(s) Tensid(e), vorzugsweise Natriumlaurylsulfat enthalten.

Ein besonders vorteilhafter Effekt auf Zahnfleisch und Mundschleimhaut ist dann zu beobachten, wenn die Mittel sowohl anionische(s) Tensid(e) und Vitamin E enthalten und die Gesamtmenge an anionischen Tensiden, die in den erfindungsgemäßen Mitteln eingesetzt wird, auf die im Mittel enthaltende Menge an Vitamin E (a) abgestimmt ist. Vorzugsweise beträgt die Menge an Aniontensiden dabei maximal das 20-fache der Menge an Vitamin E, weiter bevorzugt maximal das 18-fache, noch weiter bevorzugt maximal das 16-fache und insbesondere maximal das 15-fache der Menge an Vitamin E.

Zusätzlich zum Cocoamidopropylbetain können die erfindungsgemäßen Mund- und Zahnpflege- und -reinigungsmittel weitere(s) amphotere(s) Tensid(e) enthalten.

Amphotere Tenside werden in ampholytische Tenside und zwitterionische Tenside unterteilt. Unter ampholytischen Tensiden werden solche oberflächenaktiven Verbindungen verstanden, die sowohl saure als auch basische hydrophile Gruppen besitzen und sich also je nach Bedingung sauer oder basisch verhalten.

Geeignete ampholytische Tenside enthalten beispielsweise außer einer C₈ - C₂₄ - Alkyl- oder -Acylgruppe mindestens eine freie Aminogruppe (basische hydrophile Gruppe) und mindestens eine -COOH-oder -SO₃H-Gruppe (saure hydrophile Gruppe) und sind zur Ausbildung innerer Salze befähigt. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Besonders bevorzugte erfindungsgemäße Mund und Zahnpflege- und -reinigungsmittel sind dadurch gekennzeichnet, dass sie amphotere(s) Tensid(e) aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂ - C₁₈ - Acylsarcosin,
- N-Alkyl-N,N-dimethylammonium-glycinate, beispielsweise Kokosalkyl-dimethylammoniumglycinat,
- N-Acyl-aminopropyl-N,N-dimethylammoniumglycinate, beispielsweise Kokosacylaminopropyldimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Disodium Cocoamphodiacetate bekannten Verbindungen enthalten.

Wenn die Mitte sowohl Amphotenside als auch Vitamin E enthalten und die Gesamtmenge der im erfindungsgemäßen Mittel eingesetzten amphoteren Tenside auf die im Mittel enthaltene Menge an gegebenenfalls acetyliertem Vitamin E (a) abgestimmt ist, so ist dies ebenfalls von besonderem Vorteil im Hinblick auf die Vitalisierung von Zahnfleisch und Mundschleimhaut. Aus diesem Grund ist es bevorzugt, wenn die amphoteren Tenside - bezogen auf ihre Gesamtmenge im Mittel - in speziellen Mengenbereichen eingesetzt werden.

Die erfindungsgemäß bevorzugte Kombination aus anionischem und amphoterem Tensid ist schonend für das Zahnfleisch und stellt gleichzeitig eine ausreichende Reinigung der Zahnoberflächen sicher.

Für die Wirkung der erfindungsgemäßen Mittel hat es sich als vorteilhaft erwiesen, die zuvor beschriebene Wirkstoffkombination aus Vitamin E, anionischem Tensid und amphoterem Tensid durch weitere spezifische Wirkstoffe zu ergänzen, wobei sich wundheilende und entzündungshemmende Stoffe und Fluoridverbindungen als besonderes wirkungsvoll erwiesen haben.

Als wundheilende und entzündungshemmende Stoffe eignen sich beispielsweise Allantoin, Azulen, Kamillenextrakten, Tocopherol, Panthenol, Bisabolol, insbesondere jedoch Salbeiextrakte. Mund- und Zahnpflege- und -reinigungsmittel nach einem der vorherigen Ansprüche, dadurch gekennzeichnet, dass sie, bezogen auf ihr Gesamtgewicht, 0,001 bis 2,0 Gew.-%, vorzugsweise 0,01 bis 1,0 Gew.-% und insbesondere 0,05 bis 0,25 Gew.-% Salbei-Blattextrakt enthalten, werden erfindungsgemäß bevorzugt.

Ein Extrakt im Sinne der vorliegenden Anmeldung ist ein Stoff oder Stoffgemisch, welches durch Extraktion gewonnen wurde. Die Angaben zum Gewichtsanteil des Extrakts am Gesamtgewicht erfindungsgemäßer Mittel beziehen sich also auf die aus dem Extraktionsgut durch Extraktion erhaltenen Stoffe oder Stoffgemische nicht jedoch auf etwaige Hilfs- oder Begleitstoffe, wie die zur Extraktion eingesetzten Lösungsmittel.

Man unterscheidet nach der Beschaffenheit *Trockenextrakte* d.h. bis zur Trockene eingedampfte Extrakte, *Fluidextrakte* d.h. mit Lösungsmitteln so hergestellte Extrakte, daß aus einem Teil Droge höchstens 2 Teile Fluid-Extrakt gewonnen werden, *zähflüssige Extrakte bzw. Dickextrakte,* d.h. Extrakte, bei denen ein Teil des Lösungsmittels verdampft wird.

Extrakte des Salbeis werden vor allem aus den Blättern gewonnen. Erfindungsgemäß geeignet sind sämtliche Extrakte, aus Kostengründen sind Extrakte aus den Blättern.

Die erfindungsgemäß verwendeten Extrakte werden durch Extraktion vorzugsweise mit organischen Lösemitteln, Wasser oder Gemischen daraus, gewonnen. Bevorzugt geeignete organische Lösemittel sind Ketone (z.B. Aceton), Ether, Ester, Alkohole oder halogenierte Kohlenwasserstoffe. Besonders bevorzugte Extraktionsmittel sind Wasser und/oder Alkohole. Unter den Alkoholen sind dabei (C₁ bis C₆)- Alkohole, wie Ethanol und Isopropanol und zwar sowohl als alleiniges Extraktionsmittel als auch in Mischung mit Wasser, bevorzugt. Auch solventfreie Extraktionsmethoden, einschließlich der überkritischen CO₂-Extraktion sind einsetzbar.

Mund- und Zahnpflege- und reinigungsmittel, insbesondere Zahnpasten, können zusätzlich auch Substanzen enthalten, die gegen Plaque und/oder Zahnstein wirksam sind. Hierzu eignen sich beispielsweise antimikrobielle Stoffe und Konservierungsmittel (Plaque) oder Chelatbildner (Zahnstein).

Erfindungsgemäß bevorzugte Mund- und Zahnpflege- und -reinigungsmittel sind dadurch gekennzeichnet, dass sie zusätzlich Antiplaque-Wirkstoffe, vorzugsweise Alkypyridiniumsalze, insbesondere Cetylpyridiniumchlorid, p-Hydroxybenzoesäuremethyl-, -ethyl- oder propylester, Natriumsorbat, Natriumbenzoat, Bromchlorophen, Triclosan, Hexetidine, Phenyl- Salicylsäureester, Biguanide z. B. Chlorhexidin, Thymol, vorzugsweise in Mengen von 0,05 bis 5 Gew.%, vorzugsweise von 0,10 bis 2,5 Gew.% und insbesondere von 0,30 bis 1,5 Gew.%, jeweils bezogen auf das gesamte Mittel, enthalten.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, dass sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein. Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose sowie Maltose und Dextrose.

Die erfindungsgemäßen Mittel können in einem kosmetischen, nicht therapeutischen Verfahren zur Reinigung der Zähne bei gleichzeitiger Vitalisierung bzw. Kräftigung des Zahnfleisches und der Mundschleimhaut eingesetzt werden

Erfindungsgemäße Mittel können als Zahnpasten oder Zahncremes formuliert werden.

Durch den Einsatz der erfindungsgemäßen Mittel kann die Zahnempfindlichkeit gesenkt werden, weil mikroskopische Fissuren im Zahnschmelz repariert werden. Darüber hinaus werden das Zahnfleisch und die Mundschleimhaut vitalisiert und gekräftigt.

### Beispiele:

Alle Angaben in Gew.-%.

### Zahnpastaformulierungen

| | **A** | **B** | **C** | **D** | **E** | **F** |
|---|---|---|---|---|---|---|
| Sorbitol, 70%ig | 18,5 | 30,0 | 30,0 | 18,5 | 25,0 | 19,0 |
| Glycerin | 4,0 | 5,0 | - | 6,0 | 5,0 | 3,0 |
| Calcium-Glycerophosphat | 0,2 | 0,13 | 0,15 | 0,17 | 0,13 | 0,12 |
| Carboxymethylcellulose | 0,7 | 0,6 | 0,5 | 0,6 | 0,7 | 0,8 |
| Xanthan | 0,4 | 0,4 | 0,5 | 0,5 | 0,4 | 0,4 |
| Sident^{®} 8 | 12,0 | - | 10,0 | 12,0 | 12,0 | 10,0 |
| Sident^{®} 9 | - | 10,0 | - | - | - | - |
| Sident^{®} 22S | 8,0 | 11,0 | 8,0 | 11,0 | 10,0 | 9,0 |
| Titandioxid | 0,1 | - | - | - | 0,2 | 0,1 |
| Dinatriumphosphat | 0,2 | 0,2 | - | - | - | - |
| Trinatriumphosphat | 0,3 | 0,25 | - | - | - | - |
| Natriumlaurylsulfat | 2,0 | 1,5 | 1,5 | 1,5 | 1,5 | 1,5 |
| Cocoamidopropylbetain | 0,2 | - | - | 0,15 | 0,2 | 0,25 |
| Natriumfluorid | 0,32 | 0,32 | 0,32 | 0,32 | 0,32 | 0,32 |
| Vitamin E Acetat | 0,1 | 0,1 | 0,12 | 0,11 | 0,09 | 0,1 |
| Aroma | 0,5 | 1,1 | 0,7 | 0,8 | 1,0 | 0,5 |
| Wasser | ad 100 | | | | | |

Die Formulierung A wurde an 46 Probanden verteilt, die sich über einen Zeitraum von 4 Wochen zweimal täglich mit ihr die Zähne putzten. Als Vergleich dienten die jeweils vorher vom jeweiligen Probanden benutzten handelsüblichen Zahncremes, die die erfindungsgemäße Dreierkombination nicht enthielten.

Im Anschluß an die Testzeit wurden die Probanden befragt:

| **Aussage** | **Zustimmung** |
|---|---|
| "Meine Zähne fühlen sich weniger rau an." | 28 Probanden = 61 % |
| Gereizte Stellen an meinem Zahnfleisch wurden gemildert." | 29 Probanden = 65% |
| Die Anzahl an gereizten Stellen in meinem Mund wurde verringert." | 28 Probanden = 61% |
| Die Formel ist besser geeignet für sensible Zähne und gereiztes | |
| Zahnfleisch als meine bisherige Zahncreme." | 37 Probanden = 61% |

## Patentansprüche

1. Mund- und Zahnpflege- und -reinigungsmittel, enthaltend - bezogen auf sein Gewicht -
a) 0,001 bis 5 Gew.-% Calcium-Glycerophosphat;
b) 0,1 bis 10 Gew.-% Carboxymethylcellulose;
c) 0,1 bis 10 Gew.-% Xanthan
d) 0,05 bis 5,0 Gew.-% Cocamidopropylbetain.

2. Mund- und Zahnpflege- und -reinigungsmittel nach Anspruch 1, **dadurch gekennzeichnet, daß** es 0,01 bis 2,5 Gew.-%, vorzugsweise 0,05 bis 2,0 Gew.-%, besonders bevorzugt 0,1 bis 1,0 Gew.-%, weiter bevorzugt 0,11 bis 0,75 Gew.-% und insbesondere 0,12 bis 0,5 Gew.-% Calcium-Glycerophosphat enthält.

3. Mund und Zahnpflege- und -reinigungsmittel nach einem der Ansprüche 1 oder 2, **dadurch gekennzeichnet, daß** es Calciumsalze der Glycerophosphorsäuren der Formeln (la) und (Ib) enthält HO-CH₂-CH(OH)-CH₂-OP(O)O₂²⁻ Ca²⁺ (la) HO-CH₂-CH(OP(O)O₂²⁻)-CH₂-OH Ca²⁺ (Ib).

4. Mund und Zahnpflege- und -reinigungsmittel nach Anspruch 3, **dadurch gekennzeichnet, daß** das Gewichtsverhältnis der Calciumsalze der Formeln (Ia) zu (Ib) oberhalb von 50:50, vorzugsweise oberhalb von 60:40, besonders bevorzugt oberhalb von 70:30 und insbesondere oberhalb 80:20 liegt.

5. Mund- und Zahnpflege- und -reinigungsmittel nach einem der Ansprüche 1 bis 4, **dadurch gekennzeichnet, daß** es - bezogen auf sein Gewicht - 0,2 bis 7,5 Gew.-%, vorzugsweise 0,25 bis 5 Gew.-%, besonders bevorzugt 0,3 bis 4 Gew.-%, weiter bevorzugt 0,4 bis 3 Gew.-%, noch weiter bevorzugt 0,45 bis 2 Gew.-% und insbesondere 0,5 bis 0,8 Gew.-% Carboxymethylcellulose enthält.

6. Mund- und Zahnpflege- und -reinigungsmittel nach einem der Ansprüche 1 bis 5, **dadurch gekennzeichnet, daß** es - bezogen auf sein Gewicht - 0,15 bis 5 Gew.-%, vorzugsweise 0,2 bis 2,5 Gew.-%, besonders bevorzugt 0,25 bis 1 Gew.-%, weiter bevorzugt 0,3 bis 0,75 Gew.-%, noch weiter bevorzugt 0,35 bis 0,6 Gew.-% und insbesondere 0,4 bis 0,5 Gew.-% Xanthan enthält.

7. Mund- und Zahnpflege- und -reinigungsmittel nach einem der Ansprüche 1 bis 6, **dadurch gekennzeichnet, daß** das Gewichtsverhältnis von Carboxymethylcellulose zu Xanthan bein 1:1, vorzugsweise bei ≥ 1,2:1, besonders bevorzugt bei ≥ 1,4:1 und insbesondere bei ≥ 1,5:1 liegt.

8. Mund- und Zahnpflege- und -reinigungsmittel nach einem der Ansprüche 1 bis 7, **dadurch gekennzeichnet, daß** es - bezogen auf sein Gewicht -10 bis 50 Gew.-%, vorzugsweise 12,5 bis 45 Gew.-%, besonders bevorzugt 15 bis 40 Gew.-%, weiter bevorzugt 17,5 bis 35 Gew.-% und insbesondere 20 bis 29 Gew.-% mehrwertige(n) Alkohol(e) aus der Gruppe Sorbit und/oder Glycerin und/oder 1,2-Propylenglycol.-%, jeweils bezogen auf das Gewichts des gesamten Mittels, enthält.

9. Mund- und Zahnpflege- und -reinigungsmittel nach einem der Ansprüche 1 bis 8, **dadurch gekennzeichnet, daß** es bezogen auf sein Gesamtgewicht, 0,1 bis 3,5 Gew.-%, besonders bevorzugt 0,2 bis 2,5 Gew.-% und insbesondere 0,5 bis 2,0 Gew.-% amphotere(s) Tensid(e), vorzugsweise Cocamidopropylbetain enthält.

10. Mund- und Zahnpflege- und -reinigungsmittel nach einem der Ansprüche 1 bis 9, **dadurch gekennzeichnet, daß** sie, bezogen auf ihr Gesamtgewicht, 0,05 bis 5,0 Gew.-%, vorzugsweise 0,1 bis 3,5 Gew.-%, besonders bevorzugt 0,2 bis 2,5 Gew.-% und insbesondere 0,5 bis 2,0 Gew.-% anionische(s) Tensid(e), vorzugsweise Natriumlaurylsulfat enthalten.

## Claims

1. An oral and dental care and cleaning agent, containing, based on its weight,
a) 0.001 to 5 wt.% calcium glycerophosphate;
b) 0.1 to 10 wt.% carboxymethyl cellulose;
c) 0.1 to 10 wt.% xanthan
d) 0.05 to 5.0 wt.% cocamidopropyl betaine.

2. The oral and dental care and cleaning agent according to claim 1, **characterized in that** it contains 0.01 to 2.5 wt.%, preferably 0.05 to 2.0 wt.%, particularly preferably 0.1 to 1.0 wt.%, more preferably 0.11 to 0.75 wt.% and in particular 0.12 to 0.5 wt.% calcium glycerophosphate.

3. The oral and dental care and cleaning agent according to either claim 1 or claim 2, **characterized in that** it contains calcium salts of glycerophosphoric acids of formulae (Ia) and (Ib)
HO-CH₂-CH(OH)-CH₂-OP(O)O₂²⁻ Ca²⁺ (Ia) HO-CH₂-CH(OP(O)O₂²⁻)-CH₂-OH Ca²⁺ (Ib).

4. The oral and dental care and cleaning agent according to claim 3, **characterized in that** the weight ratio of the calcium salts of formulae (Ia) to (Ib) is above 50:50, preferably above 60:40, particularly preferably above 70:30 and in particular above 80:20.

5. The oral and dental care and cleaning agent according to one of claims 1 to 4, **characterized in that** it contains, based on its weight, 0.2 to 7.5 wt.%, preferably 0.25 to 5 wt.%, particularly preferably 0.3 to 4 wt.%, more preferably 0.4 to 3 wt.%, even more preferably 0.45 to 2 wt.% and in particular 0.5 to 0.8 wt.% carboxymethyl cellulose.

6. The oral and dental care and cleaning agent according to one of claims 1 to 5, **characterized in that** it contains, based on its weight, 0.15 to 5 wt.%, preferably 0.2 to 2.5 wt.%, particularly preferably 0.25 to 1 wt.%, more preferably 0.3 to 0.75 wt.%, even more preferably 0.35 to 0.6 wt.% and in particular 0.4 to 0.5 wt.% xanthan.

7. The oral and dental care and cleaning agent according to one of claims 1 to 6, **characterized in that** the weight ratio of carboxymethyl cellulose to xanthan is ≥ 1:1, preferably ≥ 1.2:1, particularly preferably ≥ 1.4:1 and in particular ≥ 1.5:1.

8. The oral and dental care and cleaning agent according to one of claims 1 to 7, **characterized in that** it contains, based on its weight, 10 to 50 wt.%, preferably 12.5 to 45 wt.%, particularly preferably 15 to 40 wt.%, more preferably 17.5 to 35 wt.% and in particular 20 to 29 wt.% polyhydric alcohol(s) from the group consisting of sorbitol and/or glycerol and/or 1,2-propylene glycol, in each case based on the weight of the total agent.

9. The oral and dental care and cleaning agent according to one of claims 1 to 8, **characterized in that** it contains, based on its total weight, 0.1 to 3.5 wt.%, particularly preferably 0.2 to 2.5 wt.% and in particular 0.5 to 2.0 wt.% amphoteric surfactant(s), preferably cocamidopropyl betaine.

10. The oral and dental care and cleaning agent according to one of claims 1 to 9, **characterized in that** it contains, based on its total weight, 0.05 to 5.0 wt.%, preferably 0.1 to 3.5 wt.%, particularly preferably 0.2 to 2.5 wt.% and in particular 0.5 to 2.0 wt.% anionic surfactant(s), preferably sodium lauryl sulfate.

## Revendications

1. Agent de nettoyage et de soins bucco-dentaires, contenant sur la base de son poids
a) de 0,001 à 5% en poids de glycérophosphate de calcium ;
b) de 0,1 à 10% en poids de carboxyméthylcellulose ;
c) de 0,1 à 10% en poids de xanthane
d) de 0,05 à 5,0% de cocamidopropylbétaïne.

2. Agent de nettoyage et de soins bucco-dentaires selon la revendication 1, **caractérisé en ce qu'**il contient de 0,01 à 2,5% en poids, de préférence de 0,05 à 2,0% en poids, de manière particulièrement préférée de 0,1 à 1,0% en poids, de manière tout particulièrement préférée de 0,11 à 0,75% en poids et notamment de 0,12 à 0,5% en poids de glycérophosphate de calcium.

3. Agent de nettoyage et de soins bucco-dentaires selon l'une des revendications 1 ou 2, **caractérisé en ce qu'**il contient des sels de calcium des acides glycérophosphoriques des formules (Ia) et (Ib)
HO-CH₂-CH(OH)-CH₂-OP(O)O₂² Ca²⁺ (la) HO-CH₂-CH(OP(O)O₂²⁻)-CH₂-OH Ca²⁺ (Ib).

4. Agent de nettoyage et de soins bucco-dentaires selon la revendication 3, **caractérisé en ce que** le rapport en poids des sels de calcium des formules (Ia) à (Ib) est supérieur à 50:50, de préférence supérieur à 60:40, de manière particulièrement préférée supérieur à 70:30 et en particulier supérieur à 80:20.

5. Agent de nettoyage et de soins bucco-dentaires selon l'une des revendications 1 à 4, **caractérisé en ce qu'**il contient, sur la base de son poids, de 0,2 à 7,5% en poids, de préférence de 0,25 à 5% en poids, de manière particulièrement préférée de 0,3 à 4% en poids, plus préférablement de 0,4 à 3% en poids, encore plus préférablement de 0,45 à 2% en poids et en particulier de 0,5 à 0,8% en poids, de carboxyméthylcellulose.

6. Agent de nettoyage et de soins bucco-dentaires selon l'une des revendications 1 à 5, **caractérisé en ce qu'**il contient, sur la base de son poids, de 0,15 à 5% en poids, de préférence de 0,2 à 2,5% en poids, de façon particulièrement préférée de 0,25 à 1% en poids, de façon tout particulièrement préférée de 0,3 à 0,75% en poids, encore plus préférablement de 0,35 à 0,6% en poids et en particulier de 0,4 à 0,5% en poids de gomme xanthane.

7. Agent de nettoyage et de soins bucco-dentaires selon l'une des revendications 1 à 6, **caractérisé en ce que** le rapport en poids de carboxyméthylcellulose au xanthane est ≥ 1:1, de préférence ≥ 1,2:1, plus préférablement ≥ 1,4:1 et en particulier ≥ 1,5:1.

8. Agent de nettoyage et de soins bucco-dentaires selon l'une des revendications 1 à 7, **caractérisé en ce qu'**il contient, sur a base de son poids, de 10 à 50% en poids, de préférence de 12,5 à 45% en poids, de manière particulièrement préférée de 15 à 40% en poids, de préférence de 17,5 à 35% en poids et en particulier de 20 à 29% en poids d'un ou de plusieurs alcools polyvalents du groupe du sorbitol et/ou du glycérol et/ou du 1,2-propylène-glycol, à chaque fois par rapport au poids de tout l'agent.

9. Agent de nettoyage et de soins bucco-dentaires selon l'une des revendications 1 à 8, **caractérisé en ce qu'**il contient, sur la base de son poids total, de 0,1 à 3,5% en poids, de manière particulièrement préférée de 0,2 à 2,5% en poids et en particulier de 0,5 à 2,0% en poids, d'un ou de plusieurs tensioactifs amphotères, de préférence de la cocamidopropylbétaïne.

10. Agents de nettoyage et de soins bucco-dentaires selon l'une des revendications 1 à 9, **caractérisé en ce qu'**ils contiennent, sur la base de leur poids total, de 0,05 à 5,0% en poids, de 0,1 à 3,5% en poids, de préférence, de manière particulièrement préférée de 0,2 à 2,5% en poids et en particulier de 0,5 à 2,0% en poids d'un ou de plusieurs tensioactifs anioniques, de préférence du laurylsulfate de sodium.
